# EUROPÄISCHE PATENTSCHRIFT

(11) **EP 1 268 718 B1**
(45) Veröffentlichungstag und Bekanntmachung des Hinweises auf die Patenterteilung: **11.05.2005**
(21) Anmeldenummer: 01929230.9
(22) Anmeldetag: 06.03.2001
(51) Int. Cl.: C10M 123/06

(54) **VERDICKUNGSMITTEL-KOMPONENTE UND ALUMINIUMKOMPLEX-SCHMIERFETT**
THICKENER COMPONENT AND LUBRICATING GREASE CONTAINING AN ALUMINIUM COMPLEX
CONSTITUANT D'EPAISSISSANT ET LUBRIFIANT A COMPLEXE D'ALUMINIUM

(30) Priorität: 10.03.2000 DE 10011333
(43) Veröffentlichungstag der Anmeldung: 02.01.2003
(73) Patentinhaber: SASOL Germany GmbH, 20537 Hamburg (DE)
(72) Erfinder: FINMANS, Peter, 47199 Duisburg (DE); DIBLITZ, Christina, 22869 Schenefeld (DE); ALLMÜLLER, Frank, 47441 Moers (DE); HOELL, Detelf, 47441 Moers (DE)
(74) Vertreter: Schupfner, Georg U.
(86) Internationale Anmeldenummer: PCT/DE2001/000829
(87) Internationale Veröffentlichungsnummer: WO 2001/066675

(56) Entgegenhaltungen:
- EP-A- 0 029 589
- US-A- 2 768 138
- US-A- 3 345 291
- US-A- 3 591 505
- US-A- 3 620 975
- US-A- 3 791 972
- US-A- 5 358 664

## Beschreibung

Die Erfindung betrifft eine Verdickungsmittel-Komponente auf Basis einer Aluminiumcarboxylat-Verbindung sowie ein aus dieser unter Zusatz einer Basisflüssigkeit hergestelltes Aluminiumkomplex-Schmierfett.

Aluminiumkomplex-Schmierfette sind bekannt. Diese bestehen im wesentlichen aus einer mineralölischen Basisflüssigkeit und einem Verdickersystem, enthaltend eine oder mehrere Aluminiumcarboxylat-Verbindungen. Die Aiuminiumcarboxylat-Verbindung wird aus der Umsetzung einer Fettsäure und /oder einer aromatischen Carbonsäure mit einem Aluminiumalkoholat-Derivat erhalten. In der Technik eingesetzte Aluminiumalkoholate sind Aluminiumisopropoxylat und Tri-oxy-aluminium-triisopropoxid. Die Umsetzung folgt in der Theorie den unten dargestellten Reaktionsschemata:

Al(OR)₃ + R'-COOH + Ar-COOH + H₂O → Al(-OH)(-OCOR')(-OCOAr) + 3 ROH

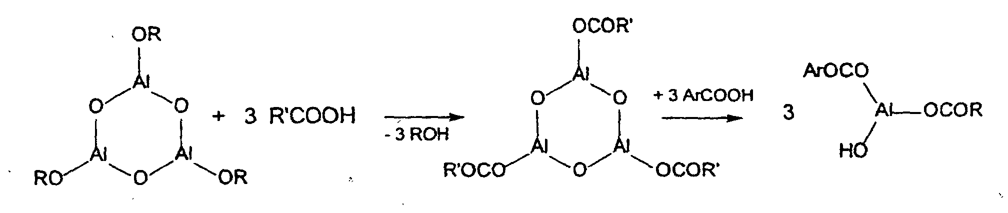

Bei Verwendung von Aluminiumisopropoxylat wird zur Bildung der in der Regel erforderlichen freien -OH Gruppe am Aluminium mit Wasser, vor, während oder nach der Umsetzung der Säuren mit dem Aluminiumalkoholat partiell hydrolisiert. Aluminiumkomplex-Schmierfette zeichnen sich durch einen hohen Tropfpunkt, gute Förderbarkeit und Beständigkeit gegen Wasser sowie niedrige Ölabscheidung aus.

Aufgabe der Erfindung ist es, wirksamere Aluminiumkomplex-Schmierfette, insbesondere geeignetere Verdickungsmittel-Komponenten zur Herstellung von Verdickem für Aluminiumkomplex-Schmierfette, bereitzustellen. Wirksamer heißt, daß das Schmierfett gegenüber herkömmlichen Aluminiumkomplex-Schmierfetten verbesserte Schmiereigenschaften aufweist. Die zur Herstellung der Aluminiumkomplex-Schmierfette eingesetzte Verdickungsmittel-Komponente soll zudem als solches lager- und verkaufsfähig sein, so daß die eigentliche Herstellung des Schmierfettes bzw. des Verdickers im Schmierfett beim Fetthersteller erfolgen kann.

Diese Aufgabe wird erfindungsgemäß gelöst durch eine Verdickungsmittel-Komponente enthaltend:
(A) 99,99 bis 94 Gew.% einer Aluminiumverbindung, die herstellbar ist durch etwa equimolare Umsetzung einer hydrolisierbaren Aluminiumverbindung mit einer oder mehreren aliphatischen Monocarbonsäuren oder deren Derivaten, in Gegenwart von Wasser und ggf. einem C1- bis C40- Alkohol, unter Ausbildung einer oder mehrerer Aluminium-Verbindungen, die im Mittel pro Aluminium-Atom eine Aluminium-Carboxylat-Bindung und des weiteren eine oder mehrere Aluminium―Hydroxy- , Aluminium-Alkoholat- oder Aluminium-Sauerstoff-Aluminium-Bindungen aufweisen, und
(B) 0,01 bis 6 Gew.% einer Esterverbindung mit 6 bis 60 Kohlenstoffatomen,
jeweils bezogen auf die Summe der Komponenten (A) und (B).

Hydrolisierbare Aluminiumverbindungen im Sinne der Erfindung sind Aluminiumverbindungen, die geeignet sind, mit protonenabgebenden Verbindungen, wie Wasser oder organischen Säuren, Aluminium-Sauerstoff-Bindungen auszubilden. Vorzugsweise sind die hydrolisierbaren Aluminiumverbindungen Aluminiumalkoholat- oder Aluminiumoxo-alkoholat-Verbindungen, wobei die Alkoholatgruppe, vorzugsweise ein C2- bis C4-Alkohol, insbesondere Isopropanolat ist. Die Carbonsäuren können sein: aliphatische verzweigte oder unverzweigte Monocarbonsäure des Typs R¹-COOH, worin R¹ ein C10-bis C40- Rest, vorzugsweise ein C 14- bis C24- Rest oder ein C 16- bis C24- Rest, ist.

Carbonsäure-Derivate im Sinne der Erfindung sind Verbindungen, die in der Lage sind, mit den Aluminiumverbindungen Aluminium-Carboxylat-Bindungen auszubilden, die auch unter Einsatz der entsprechenden Säuren entstehen würden. Genannt seien z.B. die Anhydride, Säurechloride oder Amide der oben genannten Carbonsäuren.

Ester-Verbindungen sind insbesondere solche, die sich durch Umsetzung der in der Reaktionsmischung vorhandenen Alkohole / Alkoholate mit den Carbonsäuren / Carboxylatresten bilden können.

Die Herstellung der Verdickungsmittel-Komponente erfolgt durch Umsetzung, vorzugsweise durch etwa equimolare Umsetzung (d.h. etwa 1 mol Monocarbonsäure pro mol Al-Atome in der Verbindung), einer hydrolisierbaren Aluminiumverbindung mit einer oder mehreren der oben beschriebenen Carbonsäuren oder Carbonsäure-Derivaten bei einer Temperatur, die 145 °C, besonders bevorzugt 135°C, nicht überschreitet, wobei besonders bevorzugt ein über zumindest 20°C Temperaturunterschied, vorzugsweise in einem Zeitintervall von mindestens 90 min, ansteigendes Temperaturprofil, eingehalten wird.. Vorzugsweise wird die hydrolisierbare Aluminiumverbindung vorgelegt.

Weiterhin vorzugsweise wird in einer Basisflüssigkeit umgesetzt und die bei der Umsetzung freiwerdende flüchtige Verbindung, z.B. der Alkohol, dem Gleichgewicht entzogen.

Verdicker im Sinne der Erfindung ist ein aus der Verdickungsmittel-Konponente hergestellte Verbindung bzw. Verbindungsgemisch, das durch Umsetzung der Verdickungsmittel-Konponente mit aromatischen oder cyclischen Monocarbonsäuren des Typs R²-COOH, worin R² ein C6- bis C16- Rest ist, oder deren Derivate, herstellbar ist. Die eingesetzten (aromatischen oder cyclischen) Carbonsäure-Derivate haben die oben angegebene Bedeutung.

Hierbei weist die resultierende Aluminium-Verbindung, bezogen auf die Anzahl der Carboxylatgruppen (100 mol% = alle Carboxylatgruppen), vorzugsweise zu mehr als 40 mol% aliphatische Monocarbonsäurereste auf. Der verbleibende Anteil wird von aromatischen bzw. cyclischen Monocarbonsäure-Resten gebildet.

Eine aromatische Monocarbonsäure im Sinne der Erfindung ist eine Carbonsäure, die zumindest einen Benzol-Ring oder kondensierten Benzol-Ring umfaßt und daneben aber aliphatische Kohlenwasserstoff-Reste enthalten kann. Ausdrücklich eingeschlossen sind somit auch Verbindungen wie C₆H₅-CH₂-CH₂-COOH oder CH₃-CH₂-C₆H₄- COOH.

Zur Herstellung des Verdickers als Bestandteil des Aluminiumkomplex-Schmierfettes wird dann die Verdickungsmittel-Komponente, ggf. in weiterer Basisflüssigkeit aufgenommen, mit der aromatischen bzw. cyclischen Monocarbonsäure versetzt.

Das erfindungsgemäße Herstellungsverfahren hat zur Folge, daß ein esterarmer Rohstoff (Verdickungsmittel-Komponente) zur Herstellung des eigentlichen Verdickers erhalten wird, der auch nach Lagerung esterarm bleibt.

Die erfindungsgemäßen Aluminiumkomplex-Schmierfette enthalten neben den erfindungsgemäßen Verdickem weiterhin eine Basisflüssigkeit, wobei die Basisflüssigkeit eine Kohlenwasserstoff-Verbindung und/oder ein Syntheseöl ist und der Gesamtzusammensetzung zu 30 bis 98 Gew.%, vorzugsweise zu 60 bis 95 Gew.%, zugesetzt ist.

Die Kohlenwasserstoff-Verbindung kann ein paraffinbasisches oder naphtenisches Mineralöl, ein Polyalphaolefin oder ein Weißöl sein. Weitere als Basisflüssigkeit geeignete synthetische Öle sind Fettsäureester, basierend auf ein- oder mehrfunktionellen Fettsäuren der Kettenlänge 8 bis 24 C-Atomen und ein- oder mehrfunktionellen Alkoholen. Weitere Bestandteile der erfindungsgemäßen Aluminiumkomplex-Schmierfette können weiterhin typische Additive sein. Beispiele geeigneter Additive können der Tabelle 1 entnommen werden.

Die erfindungsgemäßen Verdickungsmittel-Komponenten dienen als Rohstoffe zur Herstellung von Verdickem, die Bestandteil der Aluminiumkomplex-Schmierfette mit verbesserten Schmiereigenschaften sind. Sie werden vorzugsweise eingesetzt in Hochtemperaturanwendungen, in denen die erzielbaren hohen Tropfpunkte von besonderer Bedeutung sind, sowie in Zentralschmieranlagen und/oder zur Schmierung von Maschinen, die z. B. in Lebensmittel produzierenden oder verarbeitenden Prozessen eingesetzt werden.

Die nach dem Stand der Technik hergestellten Verdicker auf Basis von Aluminiumcarboxylat-Verbindungen bzw. die aus diesen hergestellten Aluminiumkomplex-Schmierfette weisen wesentlich höhere Esterkonzentrationen auf. Überraschend wurden durch das erfindungsgemäße Verfahren Zusammensetzungen mit niedrigeren Esterkonzentrationen (vorzugsweise < 6 % in der Verdickungsmittel-Komponente) und verbesserten Schmiereigenschaften zugänglich.

Vorzugsweise enthalten auch die Verdickungsmittel-Komponenten bereits 20 bis 80 Gew.%, besonders bevorzugt 30 bis 70 Gew.%, der oben bezeichneten Basisflüssigkeit.

### Versuchsbeispiele:

In Tabelle 2 sind Rheometrie -Werte (Physika UDS 200, oszillierende Messung, Deformation = 0,2 %, Frequenz = 0,1 Hz, Temperatur = 20 °C, Platte/Platte-Abstand = 1 mm) erfindungsgemäßer Aluminiumkomplex-Schmierfette dargestellt. Beispiel 4 weist hohe und Beispiel 5 (Vergleichsbeispiel) zu hohe Ester-Konzentrationen auf. Die Meßwerte basieren auf einer Verdickungsmittel-Komponente mit einem Al-Gehalt von 4,1 Gew.%. Das Lösemittel der Al-haltigen Verdickerkomponente ist identisch mit dem Grundöl, Ausgangsprodukt ist Aluminiumisopropanolat. Als aliphatische Monocarbonsäure wird ein technisches Stearinsäure-Gemisch eingesetzt.

### Synthese - Beispiel:

Unter Verwendung eines 5 1 Rührkessels, Stickstoff-Leitung, einer 30 cm Kolonne (verspiegelt, mit Raschigringen befüllt) und Rückflußkühler, werden die Edukte 1,466 mol (299,5 g ) DOROX ® D 10 (Aluminiumtriisopropylat, flüssig) und Mineralöl Sera ® 100 (500,0 g) (ca. 50 Gew.% paraffinbasisches Basisöl) vorgelegt und unter Rühren auf 97 °C (Sumpftemperatur) aufgeheizt. Die Stearinsäure wurde auf 75-80 °C erhitzt, um den flüssigen Aggregatzustand auch während der Einspeisung zu gewährleisten. Die flüssige Stearinsäure (SZ = 209 mg KOH/g) 1,466 mol (393,5 g) und die Mischung aus Wasser und 2-Propanol 1,372 mol (24,7g) Wasser und 2-Propanol (123,6 g) wurden innerhalb von 2,5 Stunden gleichmäßig und zeitlich parallel über getrennte Tauchrohre zudosiert und freigesetztes 2-Propanol wurde über Kopf abgezogen (Kopftemperatur max. 85 °C). Die Sumpftemperatur wird als Rampe innerhalb der 2,5 h gleichmäßig auf 127 °C erhöht. Bei einer Sumpftemperatur von 127 °C wird der Druck innerhalb von 20 min auf 200 hPa abgesenkt und Reste des Leichtsieders 2-Propanol abgezogen. Während der Umsetzung wird ein Destillat bestehend aus 3,623 mol (217,7 g) 2-Propanol aus der Reaktion und aus der Mischung (123,6 g) abgezogen und nach Reinigung und Filtration werden als Produkt ein Oxo-Aluminium-stearat gewonnen. Die Gesamtverweilzeit des Produktes bei 127 °C beträgt 30 min. (bis Ende der Vakuumphase). Die Filtration erfolgte bei etwa 120 °C durch ein 60 µm-Sieb.

Stoffparameter der Verdickungsmittel-Komponente sind in Tabelle 3 wiedergegeben.

**Tabelle 1**

| | **Konzentrations- bereich Gew.-%** | |
|---|---|---|
| Hochdruck-Additive | 2 - 10 | Dibenzyldisulfid mit chlorierten Paraffinen, geschwefelte Fettöle oder Terpene |
| Additive zur Erhöhung der Filmfestigkeit | 0,1 - 5 | Diisopropyl- oder Dilaurylhydrogenphosphit |
| Rostschutzadditive | 0,5 - 5 | Natrium-Petroleumsulfonate oder Barium-Dinonylnaphthalinsulfonat |
| Kupfer ― Desaktivatoren | 0,05 - 1 | 2-Mercaptobenzthiazol |
| Viskositätsindex-Verbesserer | 0,1 - 1 | Polymethacrylate |
| Entschlämmer | 0,001 | Siliconöle |
| Wirkstoffe zur Erzeugung fadenziehender Eigenschaften | 0,5 - 2 | Polymere |
| Anti-Verschleiß-Wirkstoffe | 0,1-2 | Trikresylphosphat, Zink - Dialkyldithiophosphate |
| Wirkstoffe zur Erzeugung wasserabstoßender Eigenschaften | 0,1 - 2 | Öl- oder andere pflanzliche Fettsäuren |
| Geruchsüberdecker | 0,05 - 0,5 | Parfüme |
| Korrosionsschutz Additive | 0,5 - 3 | Nonylphenoxyessig, Ethylendiaminsulfonat, Bleidinonylnaphthylsulfonat, Bariumsulfonate, Blei- und Zinknaphthenate |
| Oxidationsinhibitoren | | Diphenylamin, Phenyl-α-naphthylamin, Dioctyldiphenylamin, Phenothiazin, Polymeres Trimethyldihydrochinolin, 2,6-Di-tert-butyl-4-methylphenol, Bleidiamyldithiocarbamat, Dilaurylthiodipropionat-1/Citronensäure, Ascorbinsäue |

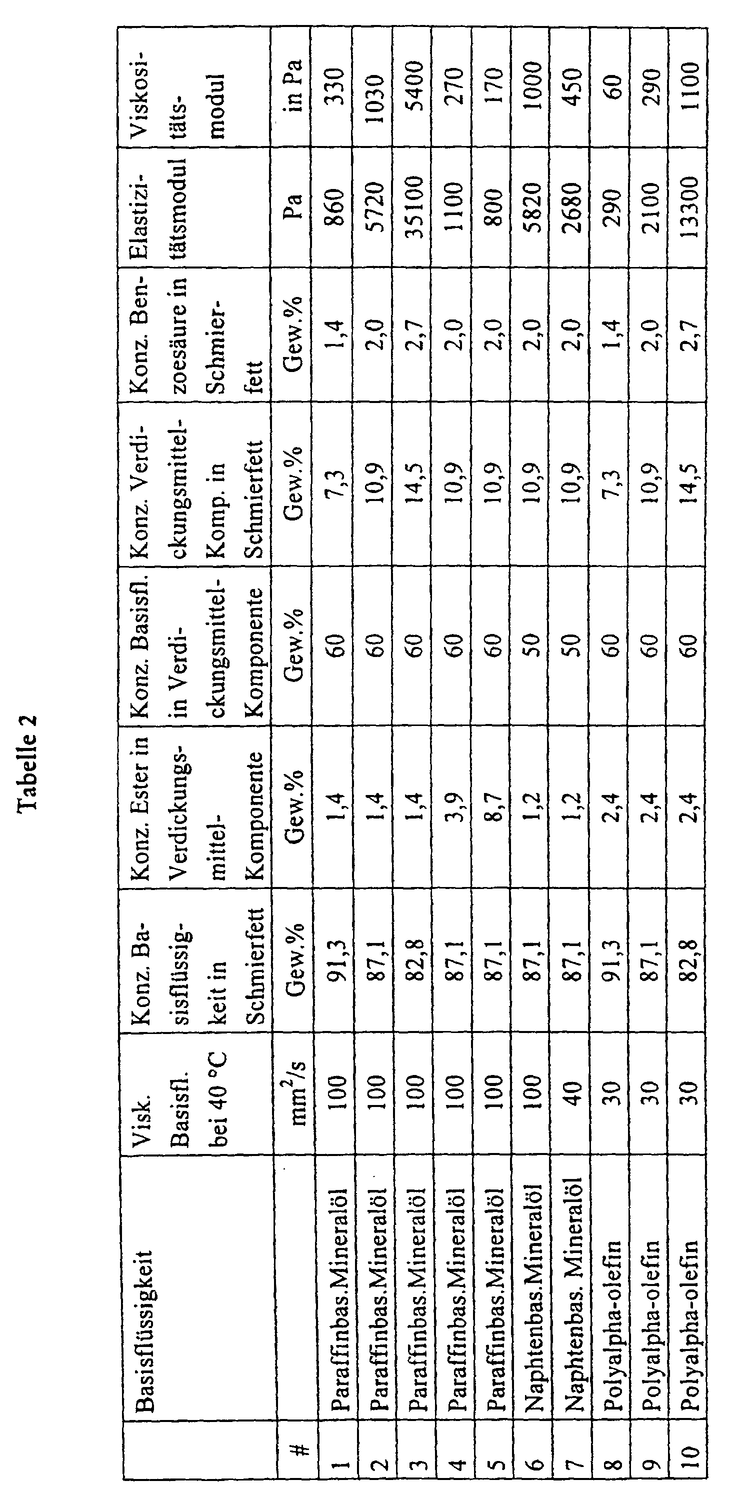

**Tabelle 3**

| | | | **Methode** |
|---|---|---|---|
| Al-Gehalt | 4,1 | % | M635 |
| Trübzahl | 20 | FNU | DIN 38404 T2 |
| Viskosität (25°C) | 1500 | mPa s | DIN 53015 |
| Viskosität (100 °C) | 100 | mPa s | Rotation (200 s⁻¹) |
| Trübzahl | 20 | FNU | DIN 38404 T2 |
| Pourpoint | 16 | °C | DIN ISO 3016 |
| Konz. 2-Propanol | 3,5 | % | |
| Dichte (20°C) | 0,94 | g /cm³ | DIN 51757 |
| Dichte (40°C) | 0,93 | g /cm³ | DIN 51757 |
| Dichte (50°C) | 0,93 | g /cm³ | DIN 51757 |
| Flammpunkt | 190 | °C | DIN 51758 |
| Farbzahl | 10 | | DIN 6162 |

## Patentansprüche

1. Verdickungsmittel-Komponente enthaltend:
(A) 99,99 bis 94 Gew.% einer Aluminiumverbindung, die herstellbar ist durch etwa equimolare Umsetzung einer hydrolisierbaren Aluminiumverbindung mit einer oder mehreren aliphatischen Monocarbonsäuren oder deren Derivaten, in Gegenwart von Wasser und ggf. einem C1- bis C40- Alkohol, unter Ausbildung einer oder mehrerer Aluminium-Verbindungen, die im Mittel pro Aluminium-Atom eine Aluminium-Carboxylat-Bindung und des weiteren eine oder mehrere Aluminium-Hydroxy- , Aluminium-Alkoholat- oder Aluminium-Sauerstoff-Aluminium-Bindungen aufweisen, und
(B) 0,01 bis 6 Gew.% einer Esterverbindung mit 6 bis 60 Kohlenstoffatomen,
jeweils bezogen auf die Summe der Komponenten (A) und (B).

2. Verdickungsmittel-Komponente gemäß Anspruch 1, **dadurch gekennzeichnet, daß** die hydrolisierbare Aluminiumverbindung eine Aluminiumalkoholat- oder Aluminium-oxo-alkoholat-Verbindung ist.

3. Verdickungsmittel-Komponente gemäß einem der vorhergehenden Ansprüche, **dadurch gekennzeichnet, daß** die aliphatischen Monocarbonsäure eine aliphatische verzweigte oder unverzweigte Monocarbonsäure des Typs R¹-COOH ist, worin R¹ ein C10- bis C40- Rest, vorzugsweise ein C14- bis C24- Rest, oder ein Derivat dieser Monocarbonsäure ist.

4. Verdickungsmittel-Komponente gemäß einem der vorhergehenden Ansprüche, **dadurch gekennzeichnet, daß** bei der Herstellung der Aluminiumverbindung durch Umsetzung einer hydrolisierbaren Aluminiumverbindung mit einer oder mehreren Carbonsäuren oder Carbonsäure-Derivaten eine Temperatur von 145°C, vorzugsweise 135°C, nicht überschritten wird.

5. Verdickungsmittel-Komponente gemäß Anspruch 4, **dadurch gekennzeichnet, daß** bei der Umsetzung ein in einem Zeitintervall von mindestens 90 min über zumindest 20°C Temperaturunterschied ansteigendes Temperaturprofil eingehalten wird.

6. Aluminiumkomplex-Schmierfett enthaltend
- eine mittels der esterarmen Verdickungsmittel-Komponente gemäß einem der vorhergehenden Ansprüche hergestellte Verbindung bzw. Verbindungsgemisch, herstellbar durch Umsetzung der VerdickunQsmittel-Komponente mit zumindest einer aromatischen und/oder cyclischen Monocarbonsäure des Typs R²-COOH. worin R² ein C6- bis C 16- Rest ist. und/oder deren Derivat, und
- weiterhin eine Basisflüssigkeit, wobei die Basisflüssigkeit eine Kohlenwasserstoff-Verbindung und/oder ein Syntheseöl ist und der Gesamtzusammensetzung zu 30 bis 98 Gew.% , vorzugsweise zu 60 bis 95 Gew.%, zugesetzt ist.

7. Aluminiumkomplex-Schmierfett gemäß Anspruch 6, **dadurch gekennzeichnet, daß** die Basisflüssigkeit eine Viskosität von 20 bis 200 mm²/s bei 40 °C gemessen nach DIN 51562 aufweist.

8. Aluminiumkomplex-Schmierfett gemäß Anspruch 6, **dadurch gekennzeichnet, daß** die Basisflüssigkeit ein Mineraloel mit einem Siedepunkt von größer 250 °C ist .

## Claims

1. A thickener component comprising
(A) from 99.99 to 94 weight percent of an aluminium compound obtainable by an approximately equimolar reaction of a hydrolysable aluminium compound with one or more aliphatic monocarboxylic acid(s) or derivatives thereof, in the presence of water and a C₁- to C₄₀-alcohol to form one or more aluminium compound(s) having on average one aluminium carboxylate bond per aluminium atom and, in addition, one or more aluminium-hydroxy-, aluminium-alcoholate-, or aluminium-oxygen-aluminium bond(s) and
(B) from 0.01 to 6 weight percent of an ester compound having from 6 to 60 carbon atoms,
each referring to the total of components (A) and (B).

2. The thickener component of claim 1,
**characterised in that** the hydrolysable aluminium compound is an aluminium alcoholate- or aluminium-oxo-alcoholate compound.

3. A thickener component according to any one of the preceding claims,
**characterised in that** the aliphatic monocarboxylic acid is a branched or unbranched aliphatic monocarboxylic acid of the type R¹-COOH, wherein R¹ is a C₁₀- to C₄₀-residue, preferably a C₁₄- to C₂₄-residue, or a derivative of said monocarboxylic acid.

4. A thickener component according to any one of the preceding claims,
**characterised in that** a temperature of 145 °C, preferably 135 °C, is not exceeded during the manufacture of the aluminium compound by reacting a hydrolysable aluminium compound with one or more carboxylic acid(s) or carboxylic acid derivative(s).

5. The thickener component of claim 4,
**characterised in that** an increasing temperature profile presenting a temperature difference of at least 20 °C is maintained at a time interval of at least 90 minutes during the reaction.

6. An aluminium complex grease comprising
- a compound or compound mixture obtainable by reacting the low in ester thickener component according to any one of the preceding claims by reaction of the thickener component with at least one aromatic and/or cyclic monocarboxylic acid, of the type
R²-COOH, wherein R² is a C6 to C16 moiety, and/or its derivative and
- in addition, a base liquid which is a hydrocarbon compound and/or a synthetic oil and which is added to the total composition in quantities of from 30 to 98 weight percent, preferably 60 to 95 weight percent.

7. The aluminium complex grease of claim 6,
**characterised in that** the base liquid has a viscosity of from 20 to 200 mm²/s at 40 °C measured in accordance with DIN 51562.

8. The aluminium complex grease of claim 6,
**characterised in that** the base liquid is a mineral oil with a boiling point of greater than 250 °C.

## Revendications

1. Composant d'épaississant contenant :
(A) 99,99 à 94 % en poids d'un composé d'aluminium qui peut être préparé par une transformation à peu près équimolaire d'un composé d'aluminium hydrolysable avec un ou plusieurs acides monocarboxyliques aliphatiques ou leurs dérivés, en présence d'eau et le cas échéant d'un alcool en C1 à C40, avec formation d'un ou de plusieurs composés d'aluminium qui présentent en moyenne par atome d'aluminium une liaison aluminium-carboxylate et en outre une ou plusieurs liaisons aluminium-hydroxy, aluminium-alcoolate ou aluminium-oxygène-aluminium, et
(B) 0,01 à 6 % en poids d'un composé ester avec 6 à 60 atomes de carbone, à chaque fois par rapport à la somme des composant (A) et (B).

2. Composant d'épaississant selon la revendication 1, **caractérisé en ce que** le composé d'aluminium hydrolysable est un composé alcoolate d'aluminium ou oxo-alcoolate d'aluminium.

3. Composant d'épaississant selon l'une quelconque des revendications précédentes, **caractérisé en ce que** l'acide monocarboxylique aliphatique est un acide monocarboxylique aliphatique ramifié ou non ramifié du type R¹-COOH, dans lequel R¹ est un radical en C10 à C40, de préférence un radical en C14 à C24, ou un dérivé dudit acide monocarboxylique.

4. Composant d'épaississant selon l'une quelconque des revendications précédentes, **caractérisé en ce que**, lors de la préparation du composé d'aluminium par transformation d'un composé d'aluminium hydrolysable avec un ou plusieurs acides carboxyliques ou dérivés d'acide carboxylique, une température de 145°C, de préférence de 135 °C, n'est pas dépassée.

5. Composant d'épaississant selon la revendication 4, **caractérisé en ce que** lors de la transformation un profil de températures montant sur une différence de températures d'au moins 20°C dans un intervalle de temps d'au moins 90 minutes, est respecté.

6. Graisse lubrifiante à base d'un complexe d'aluminium contenant
- un composé ou mélange de composés préparé au moyen du composant d'épaississant pauvre en ester selon l'une quelconque des revendications précédentes, pouvant être préparé par transformation du composant d'épaississant avec au moins un acide monocarboxylique aromatique et/ou cyclique du type R²-COOH, dans lequel R² est un radical en C6 à C16, et/ou son dérivé, et
- en outre un liquide de base qui est un composé hydrocarboné et/ou une huile de synthèse et qui est ajouté à la composition totale à raison de 30 à 98 % en poids, de préférence à raison de 60 à 95 % en poids.

7. Graisse lubrifiante à base d'un complexe d'aluminium selon la revendication 6, **caractérisée en ce que** le liquide de base présente une viscosité de 20 à 200 mm²/s mesurée à 40°C selon DIN 51562.

8. Graisse lubrifiante à base d'un complexe d'aluminium selon la revendication 6, **caractérisée en ce que** le liquide de base est une huile minérale présentant un point d'ébullition supérieur à 250°C.
